# FASCICULE DE BREVET EUROPEEN

(11) **EP 3 727 182 B1**
(45) Date de publication et mention de la délivrance du brevet: **18.12.2024**
(21) Numéro de dépôt: 18833945.1
(22) Date de dépôt: 14.12.2018
(51) Int. Cl.: A61B 50/33, A61B 46/10, A61B 50/13

(54) **EQUIPEMENT POUR LA RÉCEPTION DE MATÉRIELS CHIRURGICAUX ET/OU PRODUITS LIQUIDES**
ARTIKEL ZUR AUFNAHME CHIRURGISCHER MATERIALIEN UND/ODER FLÜSSIGER PRODUKTE
ITEM OF EQUIPMENT FOR RECEIVING SURGICAL MATERIALS AND/OR LIQUID PRODUCTS

(30) Priorité: 21.12.2017 FR 1762776
(43) Date de publication de la demande: 28.10.2020
(73) Titulaire: Lemer Pax, 44240 La Chapelle-sur-Erdre (FR)
(72) Inventeur: LEMER, Pierre-Marie, 44100 Nantes (FR)
(74) Mandataire: Jacobacci Coralis Harle
(86) Numéro de dépôt international: PCT/FR2018/053310
(87) Numéro de publication internationale: WO 2019/122637

(56) Documents cités:
- WO-A1-2014/078553
- WO-A1-96/07364
- US-A1- 2003 056 698

## Description

### DOMAINE TECHNIQUE AUQUEL SE RAPPORTE L'INVENTION

La présente invention concerne, de manière générale, le domaine des équipements pour les salles opératoires.

Elle concerne plus particulièrement un équipement pour la réception des matériels chirurgicaux et/ou produits liquides, qui sont destinés à être utilisés par un praticien au cours d'une intervention chirurgicale.

### ARRIERE-PLAN TECHNOLOGIQUE

Une salle opératoire contient l'ensemble des appareils et des matériels nécessaires à la pratique d'une intervention chirurgicale.

Cette salle opératoire contient notamment des équipements du type table roulante servant de support aux différents matériels nécessaires à l'intervention, à savoir par exemple différents contenants ou récipients, des compresses, des cathéters, des seringues, des instruments chirurgicaux (tels que des pinces, des ciseaux, etc.).

Une telle table roulante comprend un plateau qui comporte une surface supérieure plane, habituellement recouverte par un film non-tissé stérile et étanche, sur laquelle les différents matériels chirurgicaux sont destinés à être répartis.

Mais les structures actuelles de tables roulantes ne sont pas entièrement satisfaisantes.

Tout d'abord, la pose du film non-tissé sur le plateau n'est pas toujours aisée.

Également, l'aspect plan de la surface supérieure du plateau ne permet pas un positionnement et un maintien optimum des matériels.

De plus, les contenants ou récipients, utilisés pour la réception de certains liquides et instruments, sont bien souvent à usage unique et constituent un volume important de déchets qui pose ensuite des problèmes de stockage et d'élimination.

Les équipements du type table roulante sont traditionnellement positionnés derrière le praticien qui doit alors se retourner, ceci à de multiples reprises, pour avoir accès aux matériels nécessaires.

Le document WO-2014/078553 décrit un équipement qui comprend un plateau destiné à recevoir un film de surface étanche. Les moyens de placage sont donc portés par le film de surface étanche et se présentent sous la forme d'une valve.

Le document WO-96/07364 décrit un équipement qui comprend un plateau destiné à recevoir un film de surface étanche. Le film de surface étanche est réalisé dans un matériau plastique semi-rigide.

Le document US-2003/0056698 décrit une couverture stérile pour un plateau chirurgical.

Il existe par conséquent un besoin d'un nouvel équipement pour salle opératoire dont le plateau comporte une surface supérieure qui serait adaptée à un positionnement optimal du film de surface étanche, à une meilleure ergonomie dans la réception des instruments chirurgicaux, et aussi à réduire le volume de déchets générés.

### RÉSUMÉ

Afin de remédier à l'inconvénient précité de l'état de la technique, la présente invention propose un plateau pour un équipement pour la réception de matériels chirurgicaux et/ou de produits liquides selon la revendication 1 et un équipement pour la réception de matériels chirurgicaux et/ou des produits liquides pour intervention chirurgicale selon la revendication 8. Des modes de réalisation sont proposés dans les revendications dépendantes 2 à 7.

Un équipement pour la réception de matériels chirurgicaux et/ou de produits liquides est décrit, lequel équipement comprend :
- un plateau comportant une face de dessus, et
- un film de surface étanche, destiné à être rapporté de manière amovible sur ladite face de dessus dudit plateau pour former une surface supérieure stérile destinée à recevoir lesdits matériels chirurgicaux et/ou lesdits produits liquides.

Ladite face de dessus dudit plateau comporte au moins un logement ; et ledit film de surface étanche, rapporté sur ledit plateau, forme au moins une réservation qui s'étend au sein dudit au moins un logement et qui est adaptée à recevoir l'un au moins desdits matériels chirurgicaux et/ou produits liquides.

Le plateau est ainsi adapté à un positionnement optimal du film de surface étanche stérile.

De plus, le film de surface étanche est destiné à former une surface supérieure stérile qui apporte une meilleure ergonomie dans la réception des instruments chirurgicaux. Et la réservation, constituée par la partie de film associée au logement du plateau, peut permettre de remplacer un contenant ou récipient de l'état de la technique.

Selon un premier mode de réalisation particulier, le film de surface étanche consiste en un film souple destiné à épouser la face de dessus du plateau, et le plateau comporte des moyens pour le placage dudit film de surface étanche contre sa face de dessus.

Les moyens de placage comprennent avantageusement des conduits ménagés dans ledit plateau. L'un au moins desdits conduits comporte :
- une extrémité amont, débouchant au niveau de la face de dessus dudit plateau, et
- une extrémité aval, destinée à être connectée avec des moyens d'aspiration d'air,
de sorte à provoquer un placage par aspiration dudit film de surface étanche contre ladite face de dessus dudit plateau.

D'autres caractéristiques non limitatives et avantageuses de ce premier mode de réalisation, prises individuellement ou selon toutes les combinaisons techniquement possibles, sont les suivantes :
- ledit au moins un conduit est ménagé de sorte que son extrémité amont débouche dans ledit au moins un logement de la face de dessus du plateau ;
- le plateau comporte une bande de contour entourant ledit au moins un logement et au niveau de laquelle débouche l'un au moins desdits conduits ; la bande de contour consiste avantageusement en une rainure au sein de laquelle débouche ledit au moins un conduit ; la bande de contour est avantageusement bordée par au moins un joint d'étanchéité, avantageusement deux joints d'étanchéité ménagés de part et d'autre de ladite rainure ;
- plusieurs conduits débouchent au niveau de ladite bande de contour, dont les extrémités amont respectives sont réparties sur la longueur de ladite bande de contour ;
- l'équipement comporte des moyens de pilotage pour ajuster le débit d'aspiration généré par les moyens d'aspiration d'air, lesquels moyens de pilotage comportent deux configurations de fonctionnement : une configuration de fonctionnement initial, pour piloter le débit d'aspiration à une première valeur, et une configuration de fonctionnement maintien, pour piloter le débit d'aspiration à une seconde valeur, inférieure à ladite première valeur.

Toujours selon le premier mode de réalisation particulier :
- les moyens de placage comprennent avantageusement des moyens de chauffage, adaptés à provoquer une déformation dudit film de surface étanche de sorte qu'il épouse ladite face de dessus du plateau ; par exemple, le film de surface étanche consiste en un film résistif ou thermoformable autorisant un chauffage dudit film par courant électrique ;
- le film de surface étanche comporte avantageusement une bordure qui est munie d'un organe élastique et qui est destinée à venir se loger avantageusement dans une rainure annulaire,
- le film de surface étanche comprend une paroi principale, comprenant une pièce formée par une feuille souple réalisée dans un matériau intissé ou non-tissé, et des réservations, comprenant des pièces en forme de poches réalisées dans un matériau plastique.

Selon un second mode de réalisation particulier, le film de surface étanche consiste en un film semi-rigide ou en un film rigide, dans lequel ladite au moins une réservation est préformée.

De manière générale, l'équipement comporte avantageusement un piètement portant le plateau, lequel piètement comporte :
- des moyens pour le réglage en hauteur dudit plateau, et/ou
- des moyens pour la solidarisation amovible dudit piètement avec des moyens de réception complémentaires équipant une table d'opération destinée à recevoir un patient.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue générale schématique et en perspective d'un plateau destiné à équiper un équipement selon un premier mode de réalisation ;
- la figure 2 est une vue de dessus du plateau selon la figure 1 ;
- la figure 3 est une vue en coupe du plateau de la figure 2, selon un plan de coupe III-III représenté sur la figure 2, avec un film de surface étanche souple avant sa pose ;
- la figure 4 est une vue agrandie du détail IV de la figure 3, montrant la structure de la bande de contour entourant les logements du plateau ;
- la figure 5 représente le plateau de la figure 3 dont la face de dessus est recouverte par le film de surface étanche souple, pour former une surface supérieure stérile munie de réservations adaptées à recevoir les matériels chirurgicaux ;
- la figure 6 représente schématiquement un second mode de réalisation de l'équipement selon l'invention, dans lequel le plateau est destiné à être associé avec un film de surface étanche rigide/semi-rigide ;
- la figure 7 est une vue en coupe de la figure 6 qui montre, d'une part, le film de surface étanche situé au-dessus et à distance du plateau (figure 7A) et, d'autre part, le même film de surface étanche convenablement monté sur le plateau (figure 7B) ;
- la figure 8 représente une variante de réalisation pour l'équipement selon l'invention, dans laquelle l'ensemble plateau / film de surface étanche est porté par un piètement ;
- la figure 9 est une vue de côté de l'équipement selon la figure 8, pour montrer notamment les moyens pour le réglage en hauteur du plateau.

L'équipement 1 selon l'invention est destiné à équiper une salle opératoire.

Par « salle opératoire », on entend une partie d'un bloc opératoire servant à pratiquer une intervention chirurgicale sur un patient.

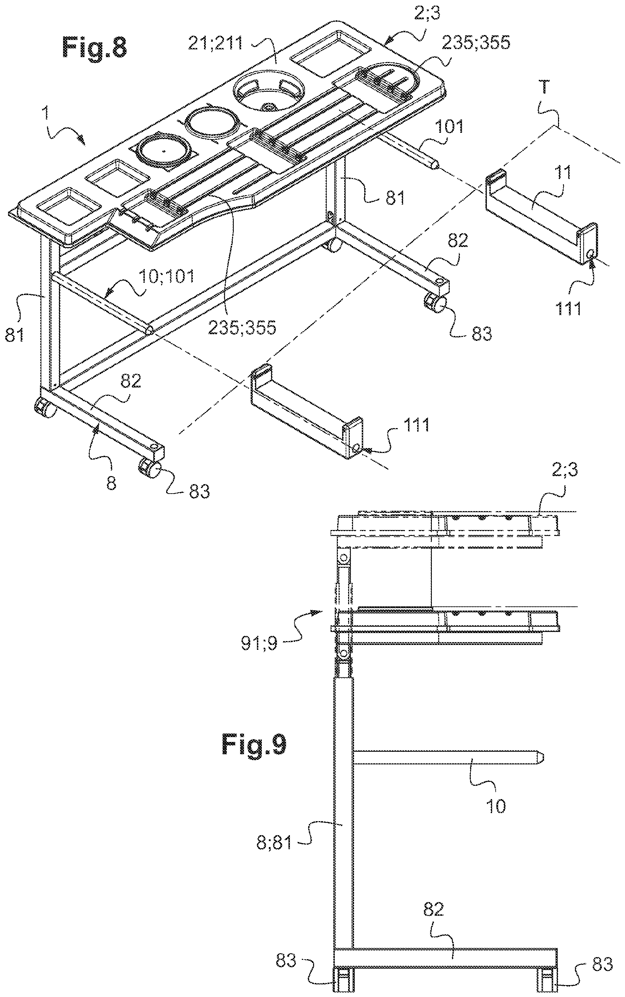

Une telle salle opératoire contient classiquement, d'une part, une table d'opération T (dont le bord est représenté très schématiquement et partiellement sur la figure 8 par le biais d'un trait discontinu), destinée à recevoir un patient et, d'autre part, l'ensemble du matériel nécessaire à chaque type d'intervention.

L'équipement 1 selon l'invention se présente avantageusement sous la forme générale d'une table roulante qui est destinée à servir de surface support pour la pose et la répartition des matériels chirurgicaux (tels que les récipients, les pinces, les ciseaux, les cathéters, les compresses, etc.) et/ou de produits liquides.

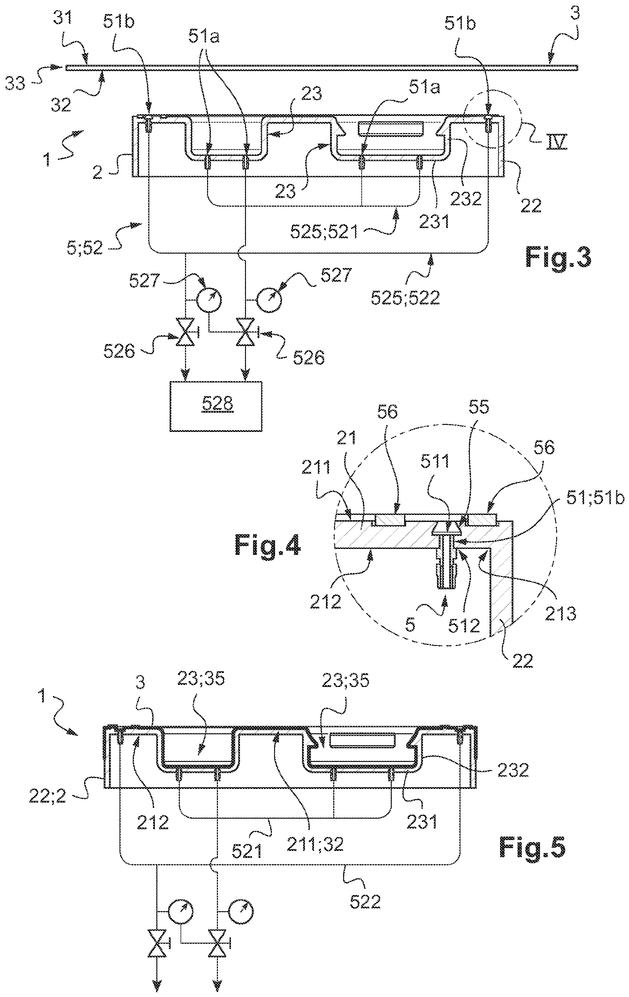

De manière générale, l'équipement 1 comprend deux principaux éléments :
- un plateau 2, servant de structure porteuse, et
- un film de surface étanche 3 (représenté sur les figures 3, 6 et 7), destiné à être rapporté de manière amovible sur ledit plateau 2 pour former une surface supérieure stérile destinée à recevoir les matériels chirurgicaux et/ou les produits liquides.

Le plateau 2 consiste avantageusement en une pièce de forme générale parallélépipédique, ou encore en forme générale de panneau ou de plaque.

Ce plateau 2 comporte une paroi principale 21 qui comporte deux faces opposées : une face de dessus 211, formant la face de dessus du plateau 2, et une face de dessous 212.

Cette paroi principale 21 comporte encore un bord périphérique 213, correspondant au bord / contour du plateau 2.

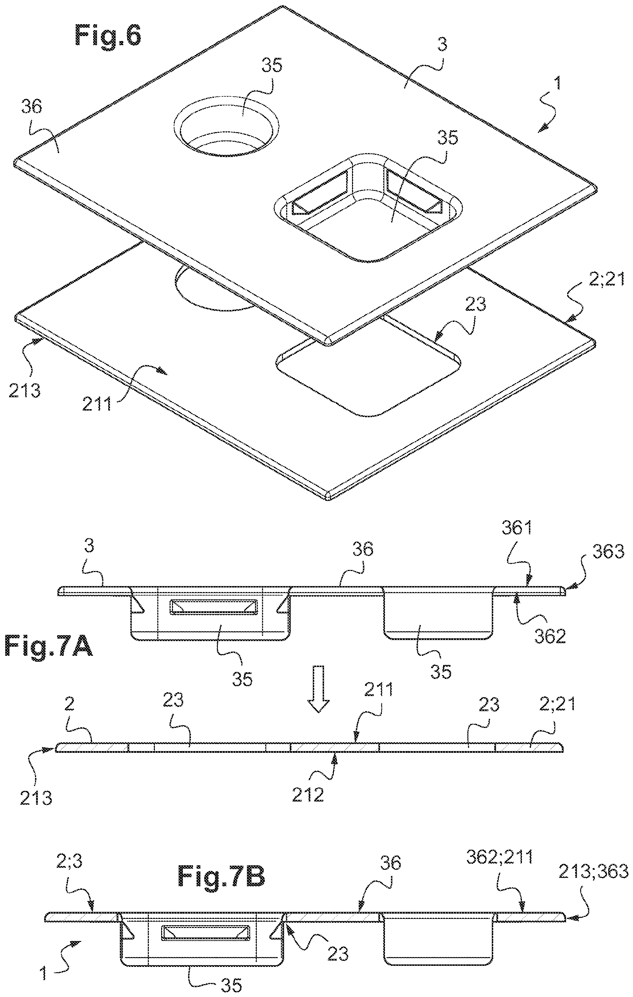

Selon l'invention, le plateau 2 et le film de surface étanche 3 sont complémentaires pour recevoir les matériels chirurgicaux et/ou les produits liquides :
- la face de dessus 211 du plateau 2 comporte des logements 23, et
- le film de surface étanche 3, rapporté sur ce plateau 2, forme des réservations 35 qui s'étendent au sein de ces logements 23 et qui sont adaptées à recevoir les matériels chirurgicaux et/ou les produits liquides (figures 5 et 7).

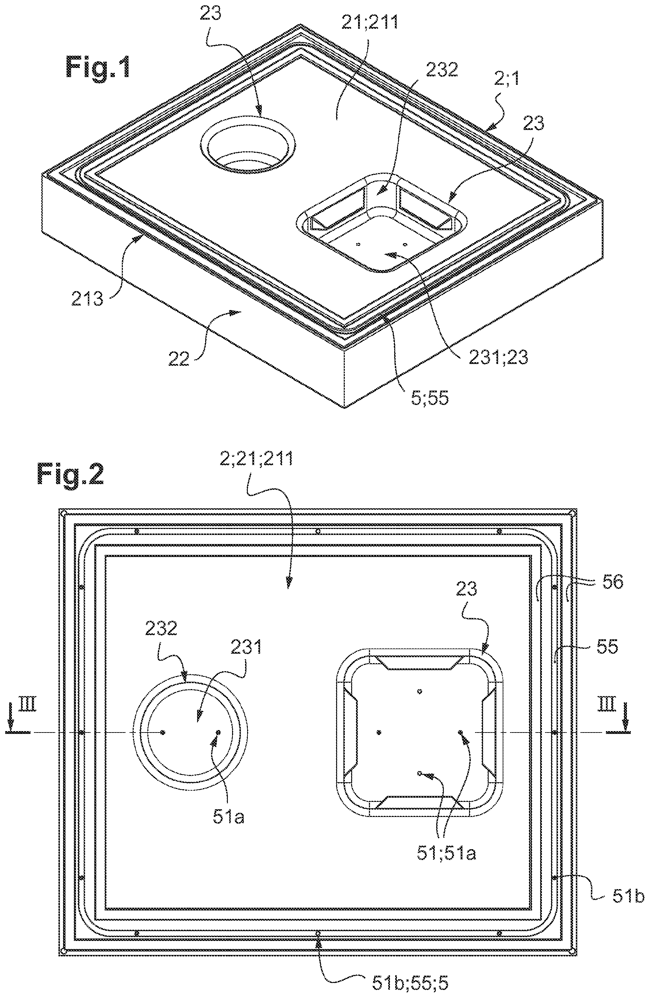

Dans un premier mode de réalisation représenté sur les figures 1 à 5, le plateau 2 comporte des moyens 5 pour le placage du film de surface étanche 3 contre sa face de dessus 211 du plateau 2. Le film de surface étanche 3 consiste alors en un film souple destiné à épouser la face de dessus 211 du plateau 2.

En l'espèce, le plateau 2 consiste avantageusement en une pièce monobloc, réalisée par exemple en un matériau plastique, de préférence en acrylonitrile butadiène styrène.

Le bord périphérique 213 de la paroi principale 21 du plateau 2 est ici prolongé par une jupe 22 qui s'étend du côté de la face de dessous 212 de cette paroi principale 21.

Les logements 23 du plateau 2 consistent en des trous borgnes (ou concavités) qui sont réalisés monobloc au niveau de la paroi principale 21 du plateau 2 et qui s'ouvrent vers le haut du côté de la face de dessus 211.

Les logements 23 ont ainsi une forme concave par rapport à la face de dessus 211 de la paroi principale 21. En d'autres termes, ces logements 23 font saillie du côté de la face de dessous 212 de la paroi principale 21.

Chaque logement 23 comprend ici une paroi de fond 231 et une paroi latérale 232.

Chaque logement 23 peut présenter des formes diverses, par exemple cylindrique ou parallélépipédique, tel qu'illustré à titre d'exemple sur les figures 1 et 2.

Les logements 23 ont par exemple une profondeur allant de 50 à 150 mm, avec des côtés allant de 50 à 400 mm.

Les moyens de plaquage 5 consistent ici en des moyens pour le plaquage du film de surface étanche 3 par phénomène d'aspiration d'air.

A cet effet, les moyens de plaquage 5 comprennent un ensemble de conduits 51 qui sont ménagés dans le plateau 2 et qui sont destinés à être connectés à des moyens d'aspiration d'air 52.

Comme illustré sur la figure 4, chaque conduit 51 s'étend dans l'épaisseur de la paroi principale 21 du plateau 2.

Chaque conduit 51 comporte deux extrémités :
- une extrémité amont 511, débouchant au niveau de la face de dessus 211 du plateau 2, et
- une extrémité aval 512, débouchant au niveau de la face de dessous 212 de la paroi principale 21 et destinée à être connectée aux moyens d'aspiration d'air 52.

Chaque conduit 51 a avantageusement un diamètre constant sur sa longueur, par exemple allant de 5 à 20 mm.

Ces conduits 51 sont ici répartis en deux groupes :
- des premiers conduits 51a, centraux, sont ménagés de sorte que leur extrémité amont 511 débouche dans l'un des logements 23 du plateau 2, et
- des seconds conduits 51b, périphériques, sont ménagés de sorte que leur extrémité amont 511 débouche au sein d'une bande de contour 55 entourant les logements 23.

Les premiers conduits 51a sont destinés à assurer un placage par aspiration du film de surface étanche 3 au sein des logements 23, de sorte à former les réservations 35 précitées.

Les seconds conduits 51b sont quant à eux prévus pour assurer une étanchéité périphérique entre la paroi principale 21 et le film de surface étanche 3.

Telle que représenté sur la figure 4, pour optimiser cette étanchéité, la bande de contour 55 consiste en une rainure ménagée au sein de la surface supérieure 211 du plateau 2, le long du bord périphérique 213 de la paroi principale 21.

Cette rainure 55 a ici une section de forme générale trapézoïdale avec, d'une part, sa grande base inférieure au sein de laquelle débouche les seconds conduits 51b et, d'autre part, sa petite base supérieure débouchant au niveau de la surface supérieure 211 du plateau 2.

Cette section particulière a pour intérêt d'assurer une étanchéité et un maintien optimum du film de surface étanche 3 contre le plateau 2.

Plusieurs seconds conduits 51b débouchent au niveau de la bande de contour 55, dont les extrémités amont 511 sont réparties sur la longueur de cette dernière (de manière adaptée pour obtenir l'étanchéité recherchée).

Par exemple, cette bande de contour 55 présente les dimensions suivantes :
- une largeur de sa grande base de 10 à 30 mm,
- une largeur de sa petite base de 5 à 20 mm, et
- une profondeur de 5 à 15 mm.

Cette bande de contour 55 est ici bordée par des joints d'étanchéité 56, avantageusement deux joints d'étanchéité 56 ménagés de part et d'autre de la bande de contour 55 (c'est-à-dire du côté intérieur de la bande de contour 55, pour l'un, et du côté extérieur de la bande de contour 55, pour l'autre).

Chaque joint d'étanchéité 56 se présente sous la forme d'une bande réalisée par exemple en matériau élastique, par exemple une mousse silicone.

La bande de contour 55 forme une boucle fermée qui entoure le ou les logements 23 ; et les deux joints d'étanchéité 56 forment chacun une boucle fermée qui suit les contours intérieur et extérieur de la bande de contour 55.

Chaque joint d'étanchéité 56 est saillant par rapport à la face de dessus 211 du plateau 2, de sorte à être comprimé lors du placage par aspiration du film plastique étanche 3.

Par exemple, ce joint d'étanchéité 56 présente les dimensions suivantes :
- une largeur de 10 à 20 mm,
- une hauteur en saillie de 3 à 10 mm.

Tel que représenté sur la figure 3, les moyens d'aspiration d'air 52, raccordés au conduit 51, comprennent avantageusement deux circuits :
- un premier circuit d'aspiration 521, raccordé aux premiers conduits 51a (débouchant dans les logements 23), et
- un second circuit d'aspiration 522, raccordé aux seconds conduits 51b (débouchant au sein de la bande de contour 55 en forme de rainure, dans le fond de cette dernière).

Chacun des deux circuits 521, 522 comprend avantageusement un ensemble de tubulures 525, équipé d'une vanne 526 et d'un vacuostat 527.

Les tubulures 525 de chaque circuit 521, 522 sont connectées, d'un côté amont, avec un groupe de conduits 51a ou 51b et, d'un côté aval, avec un dispositif d'aspiration d'air 528.

De manière alternative et non représentée, les tubulures 525 peuvent être remplacées par des chambres ou caissons qui sont formés de manière monobloc directement dans le plateau 2 (sous la paroi principale 21).

Le dispositif d'aspiration d'air 528 consiste par exemple en un générateur de vide pneumatique (effet venturi) ou en un générateur de vide électrique (pompe à vide).

On utilise avantageusement les moyens d'aspiration d'air qui équipent de manière classique les salles opératoires.

Les vannes 526 consistent avantageusement en des électrovannes qui sont adaptées à ajuster le débit d'aspiration généré au niveau des conduits 51a ou 51b associés.

Les vacuostats 527 sont des détecteurs de dépression qui permettent de détecter la dépression générée au sein des conduits 51. Ils délivrent un signal (électronique ou mécanique) indiquant la valeur de la dépression.

Ces vacuostats 527 interviennent avantageusement en tant que moyens de pilotage des vannes 526, pour ajuster le débit d'aspiration généré par les moyens d'aspiration d'air 5 au sein de chacun des deux circuits d'aspiration 521, 522.

En l'espèce, le vacuostat 527 équipant le second circuit 522 (raccordé aux seconds conduits 51b) est connecté à la vanne 526 du premier circuit 521 (raccordé aux premiers conduits 51a).

Le vacuostat 527 du second circuit 522 est ainsi destiné à piloter la vanne 526 du premier circuit 521, de sorte à déclencher son ouverture lorsqu'une valeur seuil de dépression est atteinte.

De même, ces vacuostats 527 assurent avantageusement deux configurations de fonctionnement :
- une configuration de fonctionnement initial, pour piloter le débit d'aspiration à une première valeur, relativement élevée, qui assure le placage optimal du film de surface étanche 3 contre la face de dessus 211 du plateau 2 (y compris au sein des logements 23), et
- une configuration de fonctionnement de maintien, pour piloter le débit d'aspiration à une seconde valeur, inférieure à ladite première valeur, qui assure le maintien en position du film de surface étanche 3 contre la face de dessus 211 du plateau 2.

Par exemple, les valeurs de vide sont les suivantes :
- en configuration de fonctionnement initial : 200 mbar à 900 mbar, et
- en configuration de fonctionnement de maintien : 200 mbar à 900 mbar.

Le film de surface étanche 3, destiné à être rapporté sur un tel plateau 2, est avantageusement réalisé en matériau polymère, à savoir Polyamide / Polyester, Polyamide / copolymère éthylène-alcool vinylique (EVOH) / Polyéthylène.

Ce film de surface étanche 3 a avantageusement une épaisseur comprise entre 75 et 150 µm.

De plus, il est avantageusement :
- résistant aux produits aseptisants,
- à mémoire de forme,
- à grande élongation, et
- sans élasticité.

Ce film de surface étanche 3 doit encore être étanche sous vide et étanche aux produits de déchets hospitaliers.

La face inférieure du film de surface étanche 3 peut être recouverte, au moins partiellement, par une couche d'un matériau conducteur électrique (autorisant un chauffage dudit film par courant électrique pour son thermoformage).

Tel que représenté sur la figure 3, ce film de surface étanche 3 comporte notamment deux surfaces opposées : une surface supérieure 31 et une surface inférieure 32.

Ce film de surface étanche 3 est encore délimité par une bordure périphérique 33 dont la forme correspond, au moins approximativement, à la bordure 213 de la paroi principale 21 du plateau 2.

Le film de surface étanche 3 peut être livré sous forme de rouleau, ou découpé au format du plateau 2 (éventuellement préformé) avantageusement conditionné dans un sachet individuel stérile.

La bordure 33 de ce film de surface étanche 3 peut être équipée d'un organe élastique de sorte à optimiser son maintien sur le plateau 2.

Dans ce cas, le plateau 2 peut éventuellement comporter une rainure annulaire au sein de laquelle est destinée à venir se loger cette bordure 33 « élastique » du film de surface étanche 3.

Dans une variante de réalisation, la bordure 33 du film souple 3 peut comporter des bandes adhésives adaptées à maintenir son positionnement sur le plateau 2 avant la mise en oeuvre des moyens d'aspiration 5.

La mise en place du film de surface étanche 3 sur le plateau 2 est décrite ci-dessous en relation avec la figure 5.

Pour effectuer cette mise en place, un opérateur commence par déposer le film de surface étanche 3 sur le plateau 2 de sorte que sa surface inférieure 32 repose sur la face de dessus 211 de la paroi principale 21.

Lors de ce positionnement, l'opérateur fait en sorte que le film de surface étanche 3 recouvre la bande de contour 55 afin de pouvoir générer une étanchéité périphérique continue.

Les moyens d'aspiration d'air 52 sont mis en fonctionnement avant, simultanément, ou après, cette dépose du film de surface étanche 3 sur le plateau 2.

En particulier, le dispositif d'aspiration d'air 528 est mis en fonctionnement, et la vanne 526 du second circuit d'aspiration 522 est ouverte.

Un phénomène de dépression est ainsi généré au sein de la bande de contour 55, par le second circuit d'aspiration 522.

Lorsqu'une valeur seuil de dépression est atteinte, le vacuostat 527 de ce second circuit périphérique 522 provoque le pilotage et l'ouverture de la vanne 526 équipant le premier circuit 521.

Cette ouverture permet ainsi de générer le phénomène d'aspiration d'air et de dépression au sein des logements 23 du plateau 2, de sorte que le film de surface étanche 3 vienne se plaquer également au sein de ces logements 23 par un phénomène dit d'« estampage sous vide ».

Le film de surface étanche 3 se déforme alors localement de sorte à épouser les parois de fond 231 et latérale 232 de chaque logement 23 (figure 5).

Ce film de surface étanche 3 forme directement les réservations 35 au sein de chacun des logements 23.

De préférence, le film de surface étanche 3 est adapté à rester à la forme de la surface supérieure 211 du plateau 2 (y compris des logements 23) si le vide se dégrade.

Ensuite, les vacuostats 527 pilotent les vannes 526 de sorte que le débit d'aspiration généré par les moyens d'aspiration d'air 52 assure le maintien du film de surface étanche 3 sur le plateau 2.

On obtient alors un couple plateau 2 / film de surface étanche 3, stérile, tel que représenté schématiquement sur la figure 5.

Le film de surface étanche 3, rapporté sur le plateau 2 et épousant sa surface de dessus 211, forme ainsi une surface supérieure stérile munie de réservations 35 qui s'étendent chacune au sein de l'un des logements 23 du plateau 2 et qui sont adaptées chacune à recevoir l'un au moins des matériels chirurgicaux (non représentés) et/ou des produits liquides.

Une fois l'opération terminée, les moyens d'aspiration peuvent être utilisés pour aspirer les liquides susceptibles de recouvrir le film de surface étanche 3 (en particulier les liquides éventuellement présents dans les réservations 35).

De même, il suffit à un opérateur d'arrêter les moyens de placage 5, puis de dissocier le film de surface étanche 3 par rapport au plateau 2 en vue de la mise en place d'un film de surface étanche 3 neuf.

Dans une variante de réalisation non représentée, complémentaire ou alternative de celle décrite dessus en relation avec les figures 1 à 5, l'équipement 1 peut être équipé de moyens de plaquage comprenant des moyens de chauffage qui sont adaptés à provoquer (ou à participer à) la déformation du film de surface étanche 3 de sorte qu'il épouse la face de dessus 211 du plateau 2.

Ces moyens de chauffage seraient en particulier adaptés à assurer une montée de température de la face de dessus 211 du plateau 2, par exemple à au moins 80°C.

Dans ce cas, le film de surface étanche 3 présenterait avantageusement des propriétés de thermoformage.

La face inférieure du film de surface étanche 3 peut être recouverte, au moins partiellement, par une couche d'un matériau conducteur électrique (autorisant un chauffage dudit film par courant électrique pour son thermoformage). L'équipement 1 comporte alors avantageusement des moyens pour l'alimentation électrique de ladite couche de matériau conducteur électrique.

Après utilisation, le film souple étanche 3 peut être replié sur lui-même et mis au rebu avec éventuellement d'autres matériels à usage unique utilisés lors de l'opération. Éventuellement le film souple 3 peut être inséré à ce moment dans une pochette étanche munie de moyens de mise sous vide, en vue d'en réduire le volume.

Dans un second mode de réalisation représenté sur les figures 6 et 7, l'équipement 1 comprend un film de surface étanche 3 qui consiste en un film semi-rigide ou en un film rigide préformé.

Cet équipement 1 est ainsi relativement similaire à celui décrit ci-dessus en relation avec les figures 1 à 5 en ce qu'il comprend un plateau 2 destiné à recevoir, de manière amovible, le film de surface étanche 3.

En l'espèce, les logements 23 se présentent sous la forme d'orifices traversants, débouchant au niveau des faces de dessus 211 et de dessous 212 de la paroi principale 21. En variante, les orifices 23 peuvent être remplacés par des logements borgnes.

Les orifices traversant 23 ont avantageusement un contour correspondant au jeu près, à celui des réservations 35 formées dans le film de surface étanche 3 rapporté.

De son côté, le film de surface étanche 3, destiné à être rapporté sur un tel plateau 2, est avantageusement réalisé en matériau plastique, à savoir par exemple en polytéréphtalate d'éthylène, polyamide / polyéthylène, polyamide / copolymère éthylène-alcool vinylique / polyéthylène.

Ce film de surface étanche 3 a par exemple une épaisseur comprise entre 75 et 150 µm.

Les réservations 35 sont ici préformées dans le film de surface étanche 3, cela par toute technique appropriée.

Ce film de surface étanche 3 comprend ainsi une paroi principale 36 au sein de laquelle sont préformées les réservations 35.

Par exemple, la paroi principale 36 se présente sous la forme d'une partie plane présentant une surface supérieure 361 et une surface inférieure 362.

Les réservations 35 consistent en des concavités qui sont réalisées monobloc dans la paroi principale 36 et qui s'ouvrent vers le haut au niveau de la surface supérieure 361.

Les réservations 35 ont ainsi une forme concave par rapport à la surface supérieure 361 de la paroi principale 36. En d'autres termes, ces réservations 35 font saillie du côté de la surface inférieure 362 de la paroi principale 36.

Chaque réservation 35 peut présenter des formes diverses, par exemple cylindrique ou parallélépipédique, tel qu'illustré à titre d'exemple sur les figures 6 et 7.

Cette paroi principale 36 est encore munie d'une bordure 363 en forme de lèvre périphérique orientée du côté de la face inférieure 362 précitée. Cette bordure 363 correspond, au jeu près, à la bordure 213 de la paroi principale 21 du plateau 2.

La mise en oeuvre de ce second mode de réalisation est illustrée par la figure 7.

Pour cela, le film de surface étanche 3 est positionné au-dessus du plateau 2, avec ses réservations 35 préformées orientées vers le plateau 2 (figure 7A).

Le film de surface étanche 3 est ensuite manoeuvré par l'opérateur de sorte que :
- les réservations 35 préformées viennent chacune s'insérer au travers de l'un des logements 23 complémentaires du plateau 2, et
- la face inférieure 362 de la paroi principale 36 du film de surface étanche 3 vienne recouvrir la face de dessus 211 de la paroi principale 21 du plateau 2.

On obtient alors un couple plateau 2 / film de surface étanche 3, tel que représenté schématiquement sur la figure 7B.

Les réservations 35 sont adaptées chacune à recevoir l'un au moins des matériels chirurgicaux (non représentés) et/ou des produits liquides.

Une fois l'opération terminée, il suffit à un opérateur de dissocier le film de surface étanche 3 par rapport au plateau 2 en vue de la mise en place d'un film de surface étanche 3 neuf.

Le film de surface étanche 3 peut comporter une ou plusieurs lignes de pliage pour limiter son encombrement avant et/ou après son utilisation.

L'équipement 1 correspondant comporte encore avantageusement des moyens qui permettent la solidarisation amovible entre le film 3 et le plateau 2. Ces moyens de solidarisation peuvent consister en des bandes adhésives judicieusement réparties sur la bordure périphérique 33 du film 3 ; on peut aussi prévoir des moyens d'aspiration dans le plateau 2, identiques ou similaires à ceux décrits ci-dessus en relation avec les figures 1 à 5.

Encore selon une forme de réalisation non représentée, le film de surface étanche 3 est réalisé par l'association de pièces réalisées dans deux matériaux différents (décrit ci-dessous en relation avec la figure 6 dans un souci de compréhension) :
- une paroi principale 36, par exemple une pièce formée par une feuille souple réalisée dans un matériau intissé ou non-tissé, et
- des réservations 35, par exemple des pièces en forme de poches souples ou rigide, réalisées dans un matériau plastique.

Les réservations 35 sont rapportées sur la paroi principale 36, par exemple par couture ou par soudure. En particulier, une bordure supérieure des réservations 35 est avantageusement solidarisée avec une bordure d'un orifice ménagé dans la paroi principale 36.

Les figures 8 et 9 représentent une variante des deux modes de réalisation décrits ci-dessus en relation avec les figures 1 à 7.

Un tel équipement 1 comporte l'ensemble plateau 2 / film de surface étanche 3 (tel que décrit dessus en relation avec les figures 1 à 7), associé ici à un piètement roulant 8.

La structure de l'ensemble plateau 2 / film de surface étanche 3 n'est pas limitative.

En particulier, certaines couples logements 235 / réservations 355 ont une forme générale de rainure(s), adaptés à la réception de cathéters.

Ce piètement 8 est réalisé par l'assemblage de profilés métalliques, notamment des montants verticaux 81 associés à des longerons 82 munies de roulettes 83.

Le piètement 8, et en particulier les montants 81, sont équipés de moyens 9 pour le réglage en hauteur du plateau 2.

Ces moyens de réglage 9 consistent par exemple en des montants télescopiques 91 qui coopèrent en translation avec les montants 81 du piètement 8 (représentés schématiquement sur la figure 9).

Ces moyens de réglage 9 permettent par exemple d'ajuster la hauteur du plateau 2 sur une hauteur de 30 cm, avec par exemple une hauteur maximale de 1,5 m.

Cette caractéristique technique peut être utile en vue de positionner le plateau 2 au-dessus d'une table d'opération T, de manière à permettre aux opérateurs de disposer du matériel opératoire juste au-dessus du patient.

Le piètement 8 peut encore être équipé de moyens de solidarisation amovible 10, pour la solidarisation amovible dudit piètement 8 avec des moyens de réception complémentaires 11 équipant la table d'opération T.

Les moyens de solidarisation amovible 10 de l'équipement 1 consistent par exemple en deux bras 101, horizontaux et parallèles l'un par rapport à l'autre, s'étendant sous le plateau 2 depuis les montants 81.

Les moyens de réception complémentaires 11 se présentent sous la forme de fourreaux 11 qui équipent la table d'opération T et qui sont destinés à recevoir par encastrement les bras 101.

Chaque fourreau 11 comporte en particulier un orifice cylindrique 111, complémentaire des bras 101 équipant les moyens de solidarisation amovible 10.

Chaque fourreau 11 coopère avantageusement avec son bras 101 par le biais d'un doigt d'indexation ou d'une vis de pression.

En pratique, le film de surface étanche 3 est convenablement rapporté sur le plateau 2 de l'équipement 1 (comme développé ci-dessus en relation avec les figures 1 à 7).

Le plateau 2 est ensuite ajusté en hauteur par le biais des moyens de réglage 9.

L'équipement 1 est ensuite manoeuvré en roulement de sorte que ses moyens de solidarisation amovible 10 viennent coopérer avec les moyens de réception complémentaires 11 équipant la table d'opération T.

L'équipement 1 porté par la table d'opération T peut être manoeuvré en montée / descente, mais aussi frontalement (avant / arrière) et/ou latéralement (gauche / droite), par les moyens de manoeuvre qui équipent la table T.

Suite à l'opération, le film de surface étanche 3 usagé peut être remplacé sur le plateau 2 par un nouveau, en vue d'une opération ultérieure.

L'équipement 1 conforme à l'invention permet d'optimiser les opérations de pose et de dépose du film étanche 3 sur le plateau associé 2 ; il peut permettre en particulier de limiter, voire de supprimer, l'usage de certains matériels ou accessoires volumineux du type contenants/récipients, habituellement nécessaires, et de réduire ainsi les coûts et les volumes des matériels et déchets.

## Revendications

1. Plateau pour un équipement (1) pour la réception de matériels chirurgicaux et/ou de produits liquides,
lequel plateau (2) comporte une face de dessus (211) qui est munie d'au moins un logement (23) et qui est destinée à recevoir de manière amovible un film de surface étanche (3) destiné à former au moins une réservation (35) pour recevoir lesdits matériels chirurgicaux et/ou produits liquides,
**caractérisé en ce que** le plateau (2) comporte des moyens (5) pour le placage dudit film de surface étanche (3) contre ladite face de dessus (211) dudit plateau (2),
lesquels moyens de placage (5) comprennent des conduits (51) ménagés dans ledit plateau (2),
**en ce que** l'un au moins desdits conduits (51) comporte :
- une extrémité amont (511), débouchant au niveau de la face de dessus (211) dudit plateau (2), et
- une extrémité aval (512), destinée à être connectée avec des moyens d'aspiration d'air (52),
de sorte à provoquer un placage par aspiration dudit film de surface étanche (3) contre ladite face de dessus (211) dudit plateau (2).

2. Plateau selon la revendication 1, **caractérisé en ce que** ledit au moins un conduit (51) est ménagé de sorte que son extrémité amont (511) débouche dans ledit au moins un logement (23) de la face de dessus (211) du plateau (2).

3. Plateau selon l'une quelconque des revendications 1 ou 2, **caractérisé en ce que** le plateau (2) comporte une bande de contour (55) entourant ledit au moins un logement (23) du plateau (2) et au niveau de laquelle débouche l'un au moins desdits conduits (51).

4. Plateau selon la revendication 3, **caractérisé en ce que** la bande de contour (55) consiste en une rainure au sein de laquelle débouche ledit au moins un conduit (51), laquelle bande de contour (55) est de préférence bordée par au moins un joint d'étanchéité (56).

5. Plateau selon l'une quelconque des revendications 1 à 4, **caractérisé en ce que** les moyens de placage (5) comprennent des moyens de chauffage, adaptés à provoquer une déformation dudit film de surface étanche (3) de sorte qu'il épouse ladite face de dessus (211) du plateau (2).

6. Plateau selon l'une quelconque des revendications 1 à 5, **caractérisé en ce que** le film de surface étanche (3) comprend :
- une paroi principale (36), comprenant une pièce formée par une feuille souple réalisée dans un matériau intissé ou non-tissé, et
- des réservations (35), comprenant des pièces en forme de poches réalisées dans un matériau plastique.

7. Plateau selon l'une quelconque des revendications 1 à 6, **caractérisé en ce qu'**il comporte un piètement (8) portant le plateau (2), lequel piètement (8) comporte :
- des moyens (9) pour le réglage en hauteur dudit plateau (2), et/ou
- des moyens (10) pour la solidarisation amovible dudit piètement (8) avec des moyens de réception complémentaires (11) équipant une table d'opération (T) destinée à recevoir un patient.

8. Equipement pour la réception de matériels chirurgicaux et/ou des produits liquides pour intervention chirurgicale, lequel équipement (1) comprend :
- un plateau (2) selon l'une quelconque des revendications 1 à 7, et
- un film de surface étanche (3), destiné à être rapporté de manière amovible sur ladite face de dessus (211) dudit plateau (2) pour former une surface supérieure stérile destinée à recevoir lesdits matériels chirurgicaux et/ou produits liquides,
lequel film de surface étanche (3), rapporté sur ledit plateau (2), forme au moins une réservation (35) qui s'étend au sein dudit au moins un logement (23) et qui est adaptée à recevoir l'un au moins desdits instruments chirurgicaux et/ou produits liquides,
lequel film de surface étanche (3) consiste en un film souple destiné à épouser la face de dessus (211) du plateau (2).

## Patentansprüche

1. Platte für eine Ausstattung (1) zur Aufnahme chirurgischer Materialien und/oder flüssiger Produkte ,
wobei die Platte (2) eine Oberseite (211) aufweist, die mit mindestens einer Kammer (23) versehen ist und die dazu bestimmt ist, in entfernbarer Weise einen Film mit dichter Oberfläche (3) aufzunehmen, der dazu bestimmt ist, mindestens eine Vertiefung (35) zu bilden, um die chirurgischen Materialien und/oder flüssigen Produkte aufzunehmen,
**dadurch gekennzeichnet, daß** die Platte (2) Mittel (5) zum Andrücken des Films mit dichter Oberfläche (3) an die Oberseite (211) der Platte (2) aufweist,
wobei die Andruckmittel (5) in der Platte (2) eingearbeitete Leitungen (51) aufweisen,
daß mindestens eine der Leitungen (51)
- ein stromaufwärtiges Ende (511), das im Bereich der Oberseite (211) der Platte (2) mündet, und
- ein stromabwärtiges Ende (512), das dazu bestimmt ist, mit Luftansaugmitteln (52) verbunden zu werden,
aufweist,
so daß ein Andrücken des Films mit dichter Oberfläche (3) an die Oberseite (211) der Platte (2) durch Ansaugen bewirkt wird.

2. Platte gemäß Anspruch 1, **dadurch gekennzeichnet, daß** die mindestens eine Leitung (51) so ausgeführt ist, daß deren stromaufwärtiges Ende (511) in die mindestens eine Kammer (23) der Oberseite (211) der Platte (2) mündet.

3. Platte gemäß einem der Ansprüche 1 oder 2, **dadurch gekennzeichnet, daß** die Platte (2) ein Umrandungsband (55) aufweist, das die mindestens eine Kammer (23) umrandet und in deren Bereich mindestens eine der Leitungen (51) mündet.

4. Platte gemäß Anspruch 3, **dadurch gekennzeichnet, daß** das Umrandungsband (55) aus einer Nut besteht, in die die mindestens eine Leitung (51) mündet, wobei das Umrandungsband (55) vorzugsweise von mindestens einer Dichtung (56) umgeben ist.

5. Platte gemäß einem der Ansprüche 1 bis 4, **dadurch gekennzeichnet, daß** die Andruckmittel (5) Heizmittel aufweisen, die dazu ausgelegt sind, eine Verformung des Films mit dichter Oberfläche (3) zu bewirken, so daß er an der Oberseite (211) der Platte (2) anliegt.

6. Platte gemäß einem der Ansprüche 1 bis 5, **dadurch gekennzeichnet, daß** der Film mit dichter Oberfläche (3)
- eine Hauptwandung (36), die ein durch eine aus einem Vliesmaterial oder nicht gewebten Material hergestellte flexible Folie gebildetes Teil aufweist, und
- Vertiefungen (35), die aus einem Plastikmaterial hergestellte taschenförmige Teile aufweisen,
aufweist.

7. Platte gemäß einem der Ansprüche 1 bis 6, **dadurch gekennzeichnet, daß** sie ein die Platte (2) tragendes Gestell (8) aufweist, wobei das Gestell (8)
- Mittel (9) zum Einstellen der Höhe der Platte (2) und/oder
- Mittel (10) zur abnehmbaren Befestigung des Gestells (8) an zusätzlichen Aufnahmemitteln (11), die einen Operationstisch (T), der dazu bestimmt ist, einen Patienten aufzunehmen, bestücken,
aufweist.

8. Ausstattung zur Aufnahme chirurgischer Materialien und/oder flüssiger Produkte für eine chirurgische Operation, wobei die Ausstattung (1)
- eine Platte (2) gemäß einem der Ansprüche 1 bis 7 und
- einen Film mit dichter Oberfläche (3), der dazu bestimmt ist, in abnehmbarer Weise auf die Oberseite (211) der Platte (2) aufgebracht zu werden, um eine sterile obere Oberfläche zu bilden, die dazu bestimmt ist, die chirurgischen Materialien und/oder flüssigen Produkte aufzunehmen,
aufweist,
wobei der auf die Platte (2) aufgebrachte Film mit dichter Oberfläche (3) mindestens eine Vertiefung (35) bildet, die sich in der mindestens einen Kammer (23) erstreckt und die dazu ausgelegt ist, mindestens eins der chirurgischen Instrumente und/oder flüssigen Produkte aufzunehmen,
wobei der Film mit dichter Oberfläche (3) aus einem flexiblen Film besteht, der dazu ausgelegt ist, an der Oberseite (211) der Platte (2) anzuliegen.

## Claims

1. A tray for an equipment (1) for receiving surgical materials and/or liquid products, wherein said tray (2) has an upper face (211) that is provided with at least one housing (23) and that is intended to removably receive the tight surface film (3) intended to form at least one recess (35) for receiving said surgical materials and/or liquid products,
**characterized in that** the tray (2) includes means (5) for sticking said tight surface film (3) against said upper face (211) of the tray (2),
wherein the sticking means (5) comprise channels (51) formed in said tray (2),
and **in that** one at least of said channels (51) comprises:
- an upstream end (511), opening to the upper face (211) of said tray (2), and
- a downstream end (512), intended to be connected with air suction means (52),
so as to cause a suction sticking of said tight surface film (3) against said upper face (211) of said tray (2).

2. The tray according to claim 1, **characterized in that** said at least one channel (51) is arranged so that its upstream end (511) opens to said at least one housing (23) of the upper face (211) of the tray (2).

3. The tray according to any one of claims 1 or 2, **characterized in that** the tray (2) includes a peripheral band (55) surrounding said at least one housing (23) of the tray (2) and to which opens one at least of said channels (51).

4. The tray according to claim 3, **characterized in that** the peripheral band (55) consists of a groove to which opens said at least one channel (51), wherein said peripheral band (55) is preferably lined with at least one sealing gasket (56).

5. The equipment according to any one of claims 1 à 4, **characterized in that** the sticking means (5) comprise heating means, adapted to cause a deformation of said tight surface film (3) so that the latter conforms said upper face (211) of the tray (2).

6. The tray according to any one of claims 1 to 5, **characterized in that** the tight surface film (3) comprises:
- a main wall (36), comprising a part formed by a flexible sheet made of an unwoven or non-woven material, and
- recesses (35), comprising parts in the form of pockets, made of a plastic material.

7. The tray according to any one of claims 1 to 6, **characterized in that** it comprises an underframe (8) carrying the tray (2), wherein said underframe (8) comprises:
- means (9) for adjusting said tray (2) in height, and/or
- means (10) for detachably fastening said underframe (8) with complementary receiving means (11) equipping an operation table (T) intended to receive a patient.

8. An equipment for receiving surgical materials and/or liquid products for a surgical procedure, wherein said equipment (1) comprises:
- a tray (2) according to any one of the claims 1 to 7, and
- a tight surface film (3), intended to be removably applied onto said upper face (211) of said tray (2) to form a sterile upper surface intended to receive said surgical materials and/or liquid products,
wherein said tight surface film (3), applied onto said tray (2), forms at least one recess (35) that extends into said at least one housing (23) and that is adapted to receive one at least of said surgical materials and/or liquid products,
wherein said tight surface film (3) consists of a flexible film intended to conform the upper face (211) of the tray (2).
